# EUROPÄISCHE PATENTSCHRIFT

(11) **EP 2 739 721 B1**
(45) Veröffentlichungstag und Bekanntmachung des Hinweises auf die Patenterteilung: **07.10.2015**
(21) Anmeldenummer: 12741348.2
(22) Anmeldetag: 01.08.2012
(51) Int. Cl.: C12N 1/00, C12N 1/16, C12N 1/18, C12Q 1/04

(54) **NÄHRMEDIUM**
NUTRIENT MEDIUM
MILIEU NUTRITIF

(30) Priorität: 01.08.2011 EP 11176196
(43) Veröffentlichungstag der Anmeldung: 11.06.2014
(73) Patentinhaber: Döhler GmbH, 64295 Darmstadt (DE)
(72) Erfinder: LÖGTENBÖRGER, Heinz-Jürgen, 64404 Bickenbach (DE); FIEBIG, Kay, 64285 Darmstadt (DE)
(74) Vertreter: Von Kreisler Selting Werner - Partnerschaft von Patentanwälten und Rechtsanwälten mbB
(86) Internationale Anmeldenummer: PCT/EP2012/065049
(87) Internationale Veröffentlichungsnummer: WO 2013/017626

(56) Entgegenhaltungen:
- EP-A1- 1 357 179
- DE-U1- 9 319 470
- US-A- 4 326 052
- US-A1- 2009 176 268
- KELLERMANN: "Instructions for use: GELRITE", INTERNET CITATION, 31. August 2006 (2006-08-31), Seiten 1-12, XP002666609, Gefunden im Internet: URL:http://www.carlroth.de/media/_de-de/us age/0039.pdf [gefunden am 2012-01-04]
- TAKANARI SAKAI ET AL: "The Influences of Growth Regulators and Culture Medium Composition on Shoot-Tip Cultures of Edible Canna", ENVIRONMENT CONTROL IN BIOLOGY, Bd. 45, Nr. 3, 1. Januar 2007 (2007-01-01) , Seiten 155-163, XP055015713, ISSN: 1880-554X, DOI: 10.2525/ecb.45.155
- B. HADELER ET AL: "Gelrite and Agar Differently Influence Cytokinin-Sensitivity of a Moss", JOURNAL OF PLANT PHYSIOLOGY, Bd. 146, Nr. 3, 1. Juni 1995 (1995-06-01), Seiten 369-371, XP055015731, ISSN: 0176-1617, DOI: 10.1016/S0176-1617(11)82071-7

## Beschreibung

Die Erfindung betrifft ein Nährmedium und seine Verwendung.

Bei der Herstellung eines großen Teils von vor allem Konsumgütern des täglichen Bedarfs wie z.B. Lebensmitteln beispielsweise in Form alkoholfreier Getränke oder Bieren muss auf eine Freiheit bzw. das Vorliegen einer als tolerierbar definierten maximalen Anzahl der entsprechenden Produkte von bzw. an stoffwechselfähigen, lebenden Mikroorganismen wie Schimmeln, Hefen, Milch- oder Essigsäurebakterien geachtet werden. Mikrobiologische Nachweissysteme für diesen Zweck sind lange bekannt.

Typische für analytische Zwecke eingesetzte Nährmedien sind gelierte, feste Nährmedien auf Basis beispielsweise von Agar, auf denen Mikroorganismen in Form von Kolonien wachsen können.

Ein weitverbreitetes Verfahren beruht auf dem deutschen Gebrauchsmuster 93 19 470.6, in dem ein Nährstoffmedium beschrieben wird, das beim Verwender aus trockenen Einzelbestandteilen bzw. einer entsprechenden Pulvervormischung angesetzt wird, auf einen pH-Wert von z.B. 4,3 bis 4,4 eingestellt wird und anschließend pasteurisiert wird. Während der Produktion des zu untersuchenden Guts werden aus dem Produktionsprozess Proben genommen und mit dem Nährmedium aufgefüllt. Das Wachstum von Mikroorganismen kann dann bei Vorhandensein in einer Probe in einem Zeitraum von etwa 1 bis 2 Tagen in einem Lichtkasten bei einer Temperatur von ungefähr 28°C beobachtet werden. Trübt sich eine Probe, ist sie durch Mikroorganismen belastet. Der Grad der Trübung gibt zugleich einen Anhaltspunkt über das Ausmaß der Belastung der Probe mit Mikroorganismen.

Nachteilig an dem bekannten Verfahren ist das relativ zeit- und arbeitsaufwendige Ansetzen des Nährmediums durch den Nutzer. Dies verlangt beim Anwender Erfahrung im Umgang mit mikrobiologischen Nährmedien und eine entsprechend Ausstattung des Labors mit Magnetrührern, Erlenmeyerkolben, pH-Metern mit Temperaturkompensation, Präzisionswagen, eine Pasteurisationsvorrichtung etc.

Durch Nichteinhaltung der Bedingungen oder Kontamination während des Ansetzens könnte das entsprechende Nährmedium in nichtoptimaler Qualität erhalten werden, was zu Schwierigkeiten beim Einsatz wie z.B. falsch positiven Befunden führen könnte.

Aufgabe der vorliegenden Erfindung war es, zumindest einige der Nachteile des Standes der Technik zu überwinden.

Gelöst wird die Aufgabe durch ein flüssiges Nährmedium enthaltend
- 10 bis 20 Gewichtsteile Hefeextrakt
- 15 bis 30 Gewichtsteile Pepton
- 35 bis 75 Gewichtsteile Mono- und Disaccharide
- bis 3 Gewichtsteile Mineralstoffe
- 0,02 bis 1 Gewichtsteile stabiler Gelbildner der so Stabil ist, dass das Produkt eine Autoklavierung beziehungsweise einen industrieüblichen UHT-Prozess übersteht sowie
- Wasser, wobei das Nahrmedium autoklaviert oder Ultrahocherhitzt wurde.

Wasser wird bevorzugt in einer Menge zwischen 100 und 2000, mehr bevorzugt 1000 bis 2000 Gewichtsteilen eingesetzt. Bevorzugt liegt die Konzentration des stabilen Gelbildners bei ≤ 0,5 Gew.-%, bevorzugt ≤ 0,1 Gew.-%, noch mehr bevorzugt ≤ 0,05 Gew.-%.

Im Gegensatz zu den Nährmedien des Standes der Technik kann das Produkt bereits beim Hersteller angesetzt werden und in "ready-to-use" Form an den Kunden geliefert werden.

Überraschenderweise ist das Produkt mit der erfindungsgemäßen Zusammensetzung über einen Zeitraum von mehr als sechs Monaten bei Lagerung bei Raumtemperatur stabil, wenn es autoklaviert oder alternativ ultrahocherhitzt wurde.

Das erfindungsgemäße Nährmedium enthält in üblicher Weise Nährstoffe für Mikroorganismen durch Beimengung von Hefeextrakt, Pepton, Mono- und Disacchariden und Mineralstoffen.

Erfindungsgemäß enthält das Nährmedium einen oder mehrere stabile Gelbildner, die in einer Menge enthalten sind, die zu einer fliesfähigen Konsistenz des Nährmediums führt. Der Gelbildner muss so stabil sein, dass das Produkt eine Autoklavierung, d.h. eine Erhitzung auf eine Temperatur von 121 °C für eine Haltezeit von minimal 5 Minuten bzw. einen industrieüblichen UHT-Prozess übersteht.

Übliche fließfähige Nährmedien enthalten beispielsweise Caragen. Dieses übersteht unter den gegebenen Bedingungen jedoch keine Autoklavierung und ist daher kein stabiler Gelbildner im Sinne der beschriebenen Erfindung. Bevorzugt wird als Gelbildner Gellan eingesetzt, aber auch andere natürliche, semisynthetische oder synthetische polymere Substanzen mit Gellan-analogen gelbildenden Eigenschaften, können eingesetzt werden.

Gellan-analoge gelbildende Eigenschaften bedeutet, dass die Substanzen in der Lage sind, eine Stabilisierung und Fixierung von in der Lösung beschriebenen Partikeln oder Teilchen zu erreichen, ohne ein signifikanten Viskositätsaufbau zu bewirken. Ein stabiler Geldbildner ist insbesondere ein solcher, der bei Einsatz in den genannten Mengen nach einer Autoklavierung mit einer Haltezeit von 5 min bei 121°C noch ein System bildet, dessen Viskosität über der des gleichen Systems ohne Zusatz des Gelbildners liegt, aber weniger als 100 mPa·s bei 20°C beträgt.

Bevorzugt liegt die Viskosität des Systems bei mindestens 2 mPa·s.

Gellan ist beispielsweise unter der Bezeichnung Gelrite von Merck und Co. USA erhältlich. Gellan wird im Stand der Technik so verwendet, dass es in Gegenwart von zweiwertigen Ionen eingesetzt wird und feste Gele bildet. Die erfindungsgemäßen Nährmedien sind im Gegensatz hierzu flüssig.

Nicht stabile Gelbildner, wie beispielsweise Caragen, werden beim Autoklavieren so verändert, dass sie ihre gelbildenden Eigenschaften verlieren.

Sie können zwar gegebenenfalls pasteurisiert werden, dies genügt jedoch nicht für eine langfristige Lagerfähigkeit der entsprechenden Nährmedien, sondern bedeutet, dass die Nährmedien dann kurzfristig eingesetzt werden müssen.

In den genannten Mengenbereichen ergeben sich Flüssigkeiten, die bevorzugt eine Viskosität < 100, bevorzugt < 10 mPa·s bei 20°C aufweisen. Im Gegensatz zu üblichen Nährböden, die entweder stichfest sind oder als Boullion keine Fixierung und ein Zusammenhalten einer Mikroorganismen-Kolonie gewährleisten, sind die erfindungsgemäßen Nährmedien fliesfähig und sorgen für eine Fixierung (keine Sedimentation) und den Zusammenhalt einer wachsenden Kolonie (somit gut für das menschliche Auge erkennbar). Nährstoffe können frei diffundieren ohne durch eine Matrix behindert zu werden.

Durch die erfindungsgemäße Zusammensetzung sind die Nährmedien nach Autoklavierung oder alternativ Ultrahocherhitzung über einen langen Zeitraum lagerfähig. Bei einer zu bevorzugenden dunklen Lagerung bei 4 bis 8 °C in einem verschlossenen Gefäß ändern sich die Eigenschaften des Produktes über einen Zeitraum von zwölf Monaten nicht.

Während es möglich ist, das Nährmedium als Vorratsflasche an den Anwendern zu liefern, wird es besonders bevorzugt, dass das Nährmedium portionsweise verpackt wird, beispielsweise in Gefäßen mit durchsichtigen Wandungen, die dicht verschlossen werden können. Besonders geeignet sind beispielsweise Schraubdeckelgefäße. Beim Anwender muss das Schraubdeckelgefäß nur noch geöffnet, die Probe zugegeben und das Schraubdeckelgefäß wieder verschlossen werden.

Zum Einsatz wird das Nährmedium mit einer Probe versetzt und in einem Inkubationsschrank, beispielsweise bei 25 bis 37°C gelagert. Die Mikroorganismen können im Nährmedium wachsen, was sich an einer im Minimalfall lokal begrenzten Trübung des Mediums zeigt. Die Stärke der Trübung kann zugleich ein Maß für die Keimbelastung der Probe sein. Auf diese Weise kann in einfacher Weise eine große Anzahl von Proben "auf den ersten Blick" in belastete und unbelastete Proben unterschieden werden.

Der Einsatz des Nährmediums eignet sich vor allem in Bereichen, in denen auf Kontrolle des mikrobiologischen Status von Erzeugnissen Wert gelegt wird, die von Lebewesen konsumiert oder an diesen in allen denkbaren Dareichungsformen appliziert werden, beispielsweise im Bereich der Getränke- und Lebensmittelherstellung, der Futtermittelherstellung, der Kosmetik oder der Pharmaindustrie. Zudem eignet sich das Nährmedium in fallweise adaptierter Form für den Bereich der klinischen Diagnostik.

Grundsätzlich können die Mikroorganismen aus den Nährmedien in einfacher Weise durch Pipettieren gewonnen werden und näher charakterisiert werden, beispielsweise mittels PCR oder ähnlicher Verfahren.

Die Erfindung wird durch die folgenden, nicht limitierenden Beispiele näher erläutert:

### Vergleichsbeispiel 1

Ein Nährmedium wurde mit folgenden Inhaltsstoffen in der Trockenmasse angesetzt:
70% Mono- und Disaccharide
2% Mineralstoffe
14% Hefeextrakt
15,1% Pepton
0,9% Caragen.

5 Teile dieser Trockenmasse wurden mit 95 Teilen Wasser angesetzt und das resultierende Nachweismedium nach pH-Werteinstellung auf pH = 4,3 und Pasteurisation bei 80°C für 5 Minuten mit einer stark verdünnten Hefesuspension versetzt.

Bebrütung des Schraubdeckelgefäßes bei 25°C liefert nach 1 bis 3 Tagen ein mit dem bloßen Auge interpretierbares Ergebnis, nämlich das Wachstum von Hefezellenkolonien, die als diskrete punktförmige Eintrübungen und frei in dem Nachweismedium schwebend zu erkennen sind.

### Vergleichsbeispiel 2

Es wurde wie in Vergleichsbeispiel 1 verfahren, abweichend hiervon wurde das gebrauchsfertige Nährmedium jedoch nicht pasteurisiert, sondern bei 121°C für 5 Minuten autoklaviert.

Nach Beimpfung mit Hefezellen kommt es nach 1 bis 3 Tagen zur Bildung eines Sediments, das durch die Mikroorganismen hervorgerufen wird. Eine einfache und aussagekräftige Interpretation des mikrobiologischen Ergebnisses ist hier und zudem ohne Manipulation des Schraubdeckelgefäßes nicht möglich.

### Beispiel 1

Ein von Vergleichsbeispiel 1 und 2 bezüglich des verwendeten Gelbildners abweichendes Nährmedium wurde mit folgenden Inhaltsstoffen in der Trockenmasse angesetzt:
70% Mono- und Disaccharide
2% Mineralstoffe
14% Hefeextrakt
15,1% Pepton
0,9% Gellan.

5 Teile dieser Trockenmasse wurden mit 95 Teilen Wasser angesetzt und das resultierende Nachweismedium nach pH-Werteinstellung auf pH = 4,3 und Autoklavierung bei 121°C für fünf Minuten mit einer stark verdünnten Hefesuspension versetzt.

Bebrütung des Schraubdeckelgefäßes bei 25°C liefert nach 1 bis 2 Tagen ein mit dem bloßen Auge interpretierbares Ergebnis, nämlich das Wachstum von Hefezellenkolonien, die als diskrete punktförmige Eintrübungen und frei in dem Nachweismedium schwebend zu erkennen sind.

## Patentansprüche

1. Flüssiges Nährmedium enthaltend
• 10 bis 20 Gewichtsteile Hefeextrakt
• 15 bis 30 Gewichtsteile Pepton
• 35 bis 75 Gewichtsteile Mono- und Disaccharide
• bis 3 Gewichtsteile Mineralstoffe
• 0,02 bis 1 Gewichtsteile stabiler Gelbildner, der so Stabil ist, dass das Produkt eine Autoklavierung beziehungsweise einen industrieüblichen UHT-Prozess übersteht sowie
• Wasser, wobei das Nährmedium autoklaviert oder ultrahocherhitzt wurde.

2. Nährmedium nach Anspruch 1, **dadurch gekennzeichnet, dass** die Mono- und Disaccharide ausgewählt sind aus Maltose, Dextrose, Saccharose und Fructose.

3. Nährmedium nach Anspruch 1 oder 2, **dadurch gekennzeichnet, dass** zusätzlich 1 bis 10 Teile Milchsäure oder in äquivalenten Stoffmengen deren Salze (Lactate) enthalten sind.

4. Nährmedium nach einem der Ansprüche 1 bis 3, wobei das Nährmediengel bei 4 bis 8°C mindestens 365 Tage haltbar ist.

5. Nährmedium nach einem der Ansprüche 1 bis 4, **dadurch gekennzeichnet, dass** der stabile Gelbildner Gellan ist.

6. Nährmedium nach einem der Ansprüche 1 bis 5, wobei die Viskosität bei 20°C 2 bis 100 mPa·s beträgt.

7. Nährmedium nach einem der Ansprüche 1 bis 6, wobei der Gehalt an stabilen Gelbildnern ≤ 0,5 Gew.-% beträgt.

8. Nährmedium nach einem der Ansprüche 1 bis 7, wobei der Gehalt an stabilen Gelbildnern ≤ 0,1 Gew.-% beträgt.

9. Verwendung eines Nährmediums nach einem der Ansprüche 1 bis 8 zum mikrobiologischen Screening, in der Lebensmittelindustrie wie z.B. in der Getränkeindustrie bei der Herstellung alkoholfreier Getränke und deren Grundstoffen, in der Tierfutterindustrie, in der Kosmetik- und der Pharmaindustrie sowie im Diagnostik-Bereich.

10. Verwendung eines Nährmediums nach Anspruch 9 zum mikrobiologischen Screening im Bereich des Bierbrauens.

## Claims

1. A liquid nutrient medium comprising
• 10 to 20 parts by weight of yeast extract;
• 15 to 30 parts by weight of peptone;
• 35 to 75 parts by weight of mono- and disaccharides;
• up to 3 parts by weight of minerals;
• 0.02 to 1 part by weight of stable gelling agent that is so stable that the product withstands autoclavation or an industry-standard UHT process; and
• water, the nutrient medium having been autoclaved or heated at ultrahigh temperatures.

2. The nutrient medium according to claim 1, **characterized in that** said mono- and disaccharides are selected from maltose, dextrose, sucrose and fructose.

3. The nutrient medium according to claim 1 or 2, **characterized in that** 1 to 10 parts of lactic acid or salts thereof (lactates) in equivalent molar amounts are additionally contained.

4. The nutrient medium according to any of claims 1 to 3, wherein said nutrient medium gel is storable at 4 to 8 °C for at least 365 days.

5. The nutrient medium according to any of claims 1 to 4, **characterized in that** said stable gelling agent is gellan gum.

6. The nutrient medium according to any of claims 1 to 5, wherein the viscosity at 20 °C is from 2 to 100 mPa·s.

7. The nutrient medium according to any of claims 1 to 6, wherein the content of stable gelling agents is ≤ 0.5% by weight.

8. The nutrient medium according to any of claims 1 to 7, wherein the content of stable gelling agents is ≤ 0.1% by weight.

9. Use of a nutrient medium according to any of claims 1 to 8 for microbiological screening, in the food industry, such as in the beverage industry, in the production of non-alcoholic beverages and bases thereof, in the animal fodder industry, in the cosmetics and pharmaceutical industries, and in the diagnostic field.

10. Use of a nutrient medium according to claim 9 for microbiological screening in the field of beer brewing.

## Revendications

1. Milieu nutritif liquide, contenant
• de 10 à 20 parties en poids d'extrait de levure
• de 15 à 30 parties en poids de peptone
• de 35 à 75 parties en poids de monosaccharides et de disaccharides
• jusqu'à 3 parties en poids de matières minérales
• de 0,02 à 1 partie en poids de gélifiant stable qui est stable au point que le produit supporte un autoclavage ou respectivement un procédé UHT tel qu'utilisé dans l'industrie
• de l'eau, le milieu nutritif ayant été autoclavé ou chauffé à ultra-haute température.

2. Milieu nutritif selon la revendication 1, **caractérisé en ce que** les monosaccharides et les disaccharides sont sélectionnés parmi le maltose, le dextrose, le saccharose et le fructose.

3. Milieu nutritif selon la revendication 1 ou 2, **caractérisé en ce qu'**il comprend en outre de 1 à 10 parties d'acide lactique ou de sels de l'acide lactique (lactates) en quantités de matière équivalentes.

4. Milieu nutritif selon une des revendications 1 à 3, **caractérisé en ce que** le gel de milieu nutritif peut être conservé pendant au moins 365 jours entre 4 et 8 °C.

5. Milieu nutritif selon une des revendications 1 à 4, **caractérisé en ce que** l'agent gélifiant stable est de la gomme gellane.

6. Milieu nutritif selon une des revendications 1 à 5, la viscosité étant comprise entre 2 et 100 mPa·s à 20 °C.

7. Milieu nutritif selon une des revendications 1 à 6, la teneur en agents gélifiants stables étant ≤ 0,5 % en poids.

8. Milieu nutritif selon une des revendications 1 à 7, la teneur en agents gélifiants stables étant ≤ 0,1 % en poids.

9. Utilisation d'un milieu nutritif selon une des revendications 1 à 8 pour le screening microbiologique, dans l'industrie alimentaire comme par exemple dans l'industrie des boissons lors de la préparation de boissons sans alcool et de leurs matières de base, dans l'industrie des aliments pour animaux, dans l'industrie cosmétique et pharmaceutique ainsi que dans le secteur du diagnostic.

10. Utilisation d'un milieu nutritif selon la revendication 9 pour le screening microbiologique dans le domaine du brassage de la bière.
